Europäisches Patentamt

European Patent Office

Office européen des brevets

(11) Numéro de publication: **0 096 138**

**A1**

## (12) DEMANDE DE BREVET EUROPEEN

(21) Numéro de dépôt: 82450009.4

(22) Date de dépôt: 10.06.82

(51) Int. Cl.³: **A 61 L 2/06**
**A 23 L 3/02**

(43) Date de publication de la demande:
21.12.83 Bulletin 83/51

(84) Etats contractants désignés:
DE FR

(71) Demandeur: S.A.R.L. COMMODORE INTERNATIONAL
Complexe Artisano Industriel Secteur U
F-33290 Blanquefort(FR)

(72) Inventeur: Baguet, Paul
10 avenue des Mésanges
F-33320 Le Taillan(FR)

(74) Mandataire: Thébault, Jean-Louis
Cabinet Jean-Louis Thébault 50, Cours de Verdun
F-33000 Bordeaux(FR)

(54) Dispositif de stérilisation de récipients contenant de l'eau.

(57) L'invention concerne un dispositif de stérilisation de récipients contenant de l'eau.

L'objet de l'invention est un dispositif de stérilisation de récipients contenant en partie de l'eau et/ou des solides mouillés, en particulier des biberons, caractérisé en ce qu'il comporte une enceinte isothermique (1) fermée et mise à l'atmosphère, des moyens de chauffage (5) disposés à l'intérieur de l'enceinte, des moyens (6) de régulation de la température du fluide caloporteur, en l'occurrence essentiellement de l'air, susceptibles de maintenir constamment ladite température dans une plage déterminée de températures, au moins un orifice de passage (3) ménagé dans la paroi de l'enceinte et muni d'un dispositif de fermeture amovible et un support (8) de récipients disposé à l'intérieur de l'enceinte et susceptible de permettre l'introduction et l'extraction un à un des récipients (7) ainsi que le cheminement pas à pas de ces derniers à l'intérieur de l'enceinte de manière qu'à chaque introduction d'un nouveau récipient corresponde l'extraction de celui des récipients ayant séjourné le plus longtemps dans l'enceinte.

Application notamment aux biberons.

FIG. 2

EP 0 096 138 A1

- 1 -

DISPOSITIF DE STERILISATION DE
RECIPIENTS CONTENANT DE L'EAU

La présente invention a trait à la stérilisation de récipients contenant en partie de l'eau ou éventuellement des solides mouillés et plus particulièrement, bien que non exclusivement, à la stérilisation de biberon préalablement rempli de l'eau nécessaire à la confection du biberon.

D'une façon générale, la stérilisation mise en oeuvre dans l'invention est une stérilisation de produits contenant de l'eau non sensibles à la température de 100° C et pouvant supporter des valeurs stérilisatrices efficaces calculées suivant la formule connue :

$$Fo = 10^{\left(\frac{Tl - 121,111}{Z}\right)} \times t$$

dans laquelle :

Tl = Température de stérilisation

Z = 10°C

t = nombre de minutes d'action de la chaleur.

Il existe actuellement deux procédés de stérilisation de produits contenant de l'eau :

1) Une stérilisation "douce", dite aussi "optimale", dans laquelle on recherche d'abord le degré de contamination que peut avoir le produit après fabrication et avant stérilisation (en recherchant évidemment les meilleures conditions de fabrication donnant le minimum de contamination) et à partir de laquelle on adapte la valeur de Fo la plus petite possible pour dégrader le moins possible le produit.

2) Une stérilisation "massive" qui détruit avec certitude les germes les plus résistants grâce à des valeurs stérilisatrices importantes supérieures à 8.

L'invention se rapporte à la seconde méthode et vise à obtenir à la pression atmosphérique une véritable stérilisation, la température restant en dessous de 100°C, en évitant le recours à des appareils à pression onéreux et gros consommateurs de vapeur et donc d'énergie.

L'invention sera décrite dans son application à la stérilisation de biberons bien qu'elle puisse être appliquée à la stérilisation d'autres produits comme on le verra plus loin.

Dans le cadre de cette application aux biberons, il existe actuellement trois méthodes de stérilisation des biberons :

1) Stérilisation des biberons vides ou pleins avec les accessoires dans un autoclave en bon état de marche. Ce procédé est sûr car la valeur stérilisatrice est généralement importante. Toutefois, compte tenu du coût d'un autoclave et de son utilisation, en fait seul un petit nombre de collectivités par rapport à la totalité utilise ce procédé, et aucun particulier.

2) "Stérilisation" des biberons vides avec les accessoires dans un faitout baptisé pour la circonstance "stérilisateur". Ce procédé malgré une heure de maintien de l'ébullition ne stérilise pas car on obtient une valeur stérilisatrice Fo de 0,5 environ en une heure, et les tests bactériologiques le prouvent largement. Les tests bactériologiques montrent une non stérilisation après 3 heures de maintien de l'ébullition, alors que dans la pratique le temps d'ébullition ne dépasse pas une heure.

3) "Stérilisation" des biberons vides avec les accessoires dans un bain de solution antiseptique généralement à base de chlore, pendant 90 mn selon les notices. Ce procédé est complètement inefficace dès que les germes sont un peu résistants. On ne compte plus les épidémies dans les collectivités qui l'emploient à cause de sa commodité, et de l'absence d'un moyen réellement efficace et économique.

Le but de l'invention est de proposer un dispositif capable d'assurer une réelle et efficace stérilisation des biberons et dans des conditions particulièrement économiques.

A cet effet, l'invention a pour objet un dispositif de stérilisation de récipients contenant en partie de l'eau et/ou des solides mouillés, en particulier des biberons contenant l'eau nécessaire à la confection des biberons, caractérisé en ce qu'il comporte une enceinte isothermique fermée et mise à l'atmosphère, des moyens de chauffage disposés à l'intérieur de l'enceinte, des moyens de régulation de la température à l'intérieur de l'enceinte susceptibles de maintenir constamment ladite température dans une plage déterminée de températures de façon à avoir à l'intérieur des récipients une température légèrement inférieure à 100°C, au moins un orifice de passage aménagé dans la paroi de l'enceinte et muni d'un dispositif de fermeture amovible, et un support de récipients disposé à l'intérieur de l'enceinte et susceptible de permettre l'introduction et l'extraction un à un des récipients ainsi que le cheminement pas à pas de ces derniers à l'intérieur de l'enceinte de manière qu'à chaque introduction d'un nouveau récipient corresponde l'extraction de celui des récipients ayant séjourné le plus longtemps dans l'enceinte.

Dans un tel dispositif, en prévoyant un support de récipients capable de stocker six ou huit biberons par exemple et en faisant fonctionner en permanence jour et nuit les moyens de chauffage, on peut assurer aux biberons une stérilisation absolument totale puisque le temps de séjour de chaque biberon à l'intérieur de l'enceinte thermostatée, entre le moment où il est introduit et celui où il en est extrait, est de plusieurs heures, les biberons étant prélevés dans le dispositif un à un à chaque tétée suivant un cycle tel que le dernier biberon introduit ne sera extrait qu'une fois que tous les autres biberons qui l'ont précédé dans le dispositif auront été un à un retirés.

On a ainsi l'assurance que chaque biberon extrait a passé plusieurs heures à l'intérieur du dispositif et a donc subi une stérilisation véritable et complète à la température requise pendant un temps largement supérieur à ce qui est normalement nécessaire.

D'autres caractéristiques et avantages ressortiront de la description qui va suivre d'un mode de réalisation d'un dispositif selon l'invention, description donnée à titre d'exemple uniquement et en regard des dessins annexés sur lesquels :

- Fig.1 représente une vue de dessus schématique d'un mode de réalisation d'un dispositif conforme à l'invention appliqué à la stérilisation des biberons, et

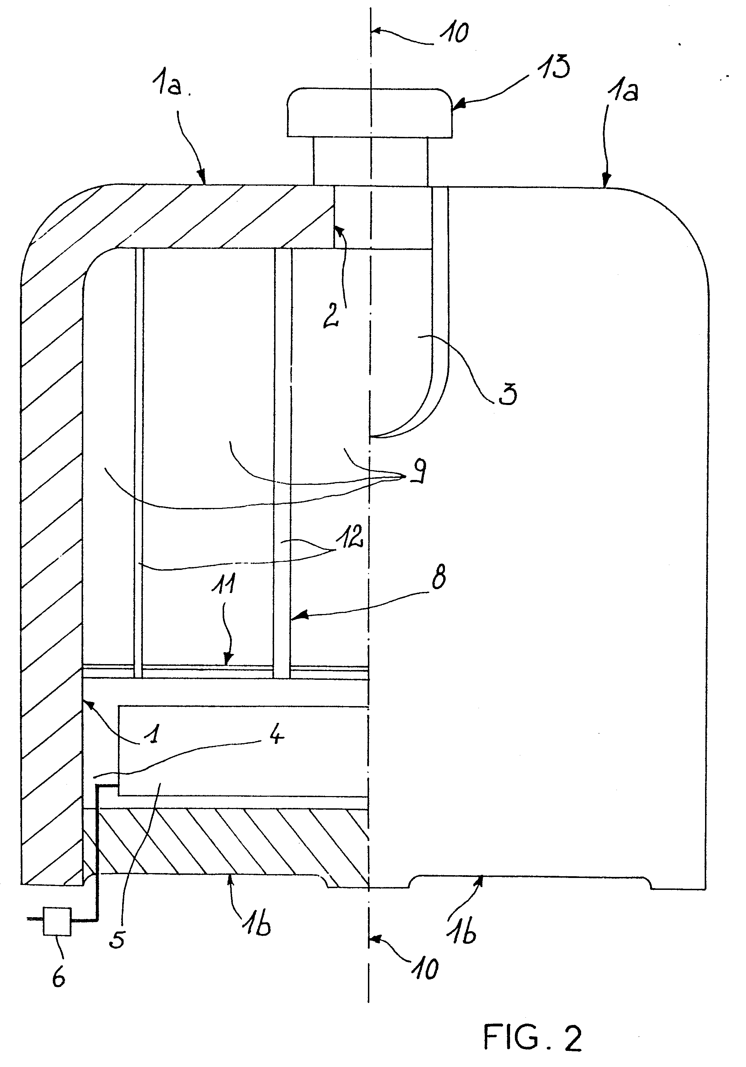

- Fig. 2 représente une vue en coupe verticale axiale partielle suivant la ligne II-II du dispositif de la Fig. 1.

Le dispositif représenté sur les dessins est constitué d'une enceinte isothermique 1 de forme générale cylindrique réalisée en un matériau de préférence ininflammable et excellent isolant thermique telle que de la laine de verre ou de roche par exemple.

L'enceinte 1 est complètement fermée excepté un passage circulaire 2 dans l'axe vertical de l'enceinte et un orifice 3 d'introduction/extraction des biberons à l'intérieur de l'enceinte. L'enceinte n'est pas toutefois isolée de l'extérieur et contient en permanence de l'air à la pression atmosphérique.

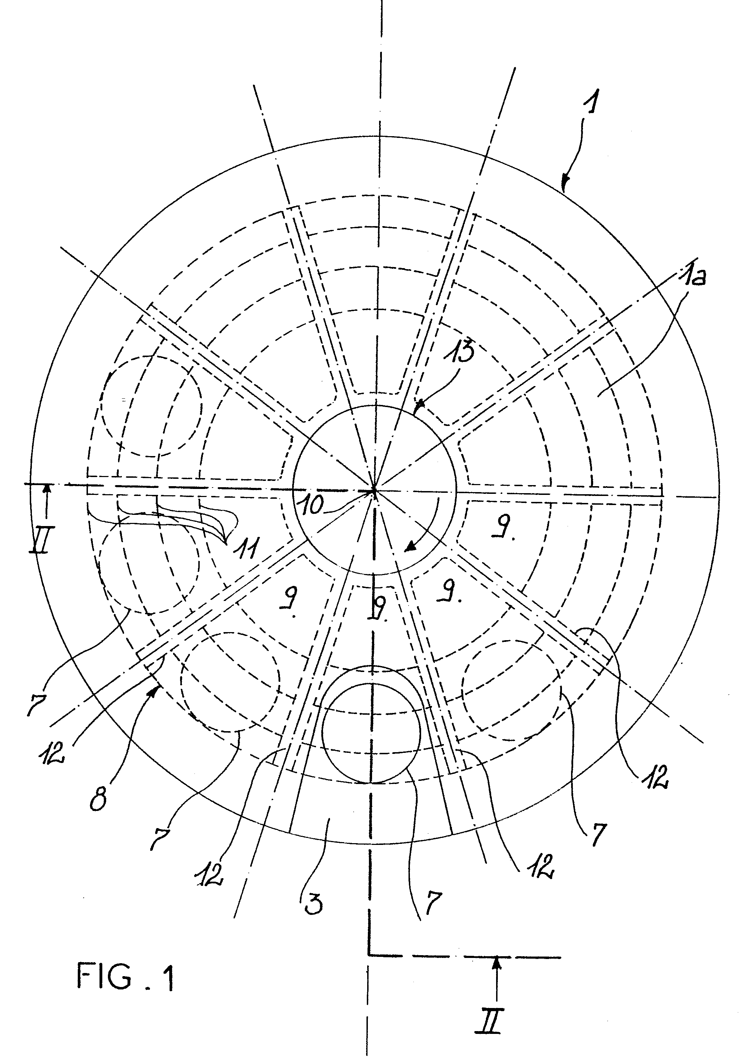

L'orifice 3 s'étend sur la partie extérieure d'un secteur angulaire (Fig. 1) correspondant à un compartiment à biberon pour ce qui concerne la paroi circulaire sommitale 1a de l'enceinte 1 et la partie supérieure correspondante de la paroi latérale cylindrique.

L'intérieur de l'enceinte 1 est divisé en deux parties, une partie inférieure 4 dans laquelle est monté un moyen de chauffage symbolisé en 5 disposé au voisinage de la paroi circulaire de fond 1b de l'enceinte 1. Ce moyen de chauffage 5 est par exemple un dispositif à résistance chauffante électrique alimenté en courant électrique par l'intermédiaire d'un dispositif de régulation de température 6 (thermostat) réglable, branché en permanence sur une source de courant électrique appropriée.

Le dimensionnement des parois de l'enceinte 1 et les caractéristiques du dispositif à résistance chauffante 5 et du régulateur 6 sont telles que le dispositif étant branché en permanence, il est possible de maintenir en permanence 24 heures sur 24 à l'intérieur de l'enceinte thermostatée 1 une température se situant à l'intérieur d'une plage relativement réduite et précise (par exemple entre 100 et 110°C correspondant à une température à l'intérieur des biberons légèrement inférieure à 100°C) avec des coûts de revient particulièrement économiques puisque pratiquement insignifiants du fait de l'excellente isolation thermique et de l'utilisation de l'air comme fluide caloporteur à l'intérieur de l'enceinte.

L'air est, en effet, le seul fluide caloporteur utilisé à l'intérieur de l'enceinte et en contact avec les parois externes des récipients à stériliser même si ces derniers sont chauffés par des parties solides ou en contact avec des parties solides, du fait de la subsistance d'une couche-limite entre les récipients et lesdites parties. L'air a une faible chaleur spécifique et sa mise en oeuvre comme fluide caloporteur est particulièrement avantageuse au niveau, d'une part,de la consommation d'énergie et, d'autre part, de la facilité et du faible coût de la régulation en température. La consommation est en fait limitée à l'énergie nécessaire pour porter le contenu des biberons de 20° à 99°C environ et pour compenser la déperdition extérieure des biberons de l'ordre de quelques watts par $m^2$ et par heure.

Au dessus de la partie 4 s'étend une chambre dans laquelle sont stockés les biberons 7 à stériliser et conserver en température. Les biberons 7 sont stockés dans une sorte de panier-magasin circulaire 8 à compartiments rayonnants 9 dans chacun desquels un biberon 7 peut être logé. Le panier-magasin 8 constitue en quelque sorte un barillet mobile autour de l'axe vertical 10 du dispositif.

A cet effet, le panier-magasin 8 est formé par exemple d'une grille circulaire de fond 11 sur laquelle reposent les biberons 7 placés verticalement, un dans chaque compartiment, la délimitation entre compartiments contigus étant faite par une paroi verticale 12 de séparation.

Le panier-magasin 8 est solidaire à sa partie supérieure d'une poignée 13 de commande de rotation, extérieure à l'enceinte 1 et reliée au panier-magasin 8.

La rigidité de l'enceinte 1 est assurée par des moyens appropriés ainsi que la suspension du panier-magasin 8 de manière que ce dernier puisse librement être tourné à la main afin de présenter devant l'orifice 3 le compartiment 9 désiré.

L'orifice 3 est obturé par un couvercle ou dispositif de fermeture amovible (non représenté) de configuration et structure appropriées.

Ce dispositif d'obturation épousant le plus étroitement possible ledit orifice 3 est seulement retiré juste pour l'introduction d'un biberon dans un compartiment 9 libre ou pour l'enlèvement d'un biberon au moment de la tétée.

Dans le mode de réalisation de la Fig. 1 il est prévu dix compartiments 9 mais ce nombre pourrait bien entendu être diminué ou au contraire augmenté.

La stérilisation des biberons (avec leur eau et leur tétine à l'intérieur) se fait à une température relativement modérée, inférieure à 100°C par rapport aux températures habituelles de stérilisation mais, étant donné le temps de séjour de plusieurs heures de chaque biberon dans le dispositif, on obtient néanmoins une stérilisation totale.

L'introduction d'un biberon se fait par l'orifice 3 en présentant un compartiment 9 vide par rotation à la main du panier-magasin 8. Ce dernier est manoeuvré toujours dans le même sens de rotation suivant la flèche de la Fig. 1 en sorte que le biberon qui vient d'être introduit n'en sera extrait qu'une fois que tous ceux qui occupent les compartiments précédents auront été retirés. Comme un biberon est retiré à chaque tétée, le temps de séjour dans le dispositif de chaque biberon est donc de plusieurs heures.

Par mesure de sécurité, avant chaque prise de biberon, le panier-magasin 8 doit être rechargé d'un nouveau biberon à stériliser. Il est intéressant de prévoir une isolation de la zone en regard de l'orifice 3 c'est-à-dire correspondant au compartiment 9 se trouvant en regard dudit orifice. Dans cette zone moins chauffée, le biberon peut se refroidir à environ 50°C pendant les trois heures par exemple qui lui restent à séjourner dans l'appareil avant son extraction pour la tétée suivante. Cela permet le mélange de l'eau chaude avec du lait en poudre froid et d'avoir un mélange aux environs de 37°C au moment de l'utilisation.

Il est à noter que le dispositif de l'invention pourrait être utilisé pour la stérilisation et/ou le maintien en température de tous récipients contenant de l'eau ou des solides mouillés. C'est ainsi que le dispositif de l'invention dans sa configuration telle que représentée sur les dessins ou dans une autre configuration, pourrait être intégré dans une chaîne de fabrication, par exemple de flacons de sérum physiologique dans laquelle les flacons une fois remplis seraient introduits, au fur et à mesure de leur fabrication, un à un dans un dispositif conforme à l'invention sans rupture de cadence. On éviterait ainsi le stockage intermédiaire et donc la recontamination entre la fabrication et la stérilisation ce qui est important pour les pyrogènes. Le conditionnement continu après la stérilisation en serait également facilité.

Dans le cadre de l'application à d'autres types de récipients que des biberons il est à souligner que la température de stérilisation à l'intérieur des récipients pourrait dépasser 100°C.

Enfin l'invention n'est évidemment pas limitée au mode de réalisation représenté et décrit ci-dessus mais en couvre au contraire toutes les variantes. C'est ainsi que les formes, dimensions, structures et configuration de l'enceinte isothermique 1 et du ou des orifices d'accès et de ses moyens d'obturation peuvent varier dans de larges mesures.

La source de chauffage 5 peut être réalisée par tous moyens appropriés, qu'ils soient électriques ou non.

Enfin, le panier-magasin 8 pourrait avoir une autre structure pourvu qu'il permette de présenter devant un ou plusieurs orifices d'introduction-extraction ménagés dans l'enceinte thermostatée, un par un les objets stérilisés ou les emplacements de réception des objets à stériliser, quelle que soit la trajectoire de déplacement. C'est ainsi que l'on peut prévoir une enceinte droite dans laquelle les biberons se suivraient les uns les autres (ou se pousseraient). Les moyens de support des biberons seraient par exemple un moyen de convoyage sans fin ou bien un simple chemin de guidage fixe dans le cas où les biberons se pousseraient les uns les autres à la queue leu leu.

R E V E N D I C A T I O N S

1. Dispositif de stérilisation de récipients contenant en partie de l'eau et/ou des solides mouillés, en particulier des biberons, caractérisé en ce qu'il comporte une enceinte isothermique (1) fermée et mise à l'atmosphère, des moyens de chauffage (5) disposés à l'intérieur de l'enceinte, des moyens (6) de régulation de la température du fluide caloporteur, en l'occurrence essentiellement de l'air, susceptibles de maintenir constamment ladite température dans une plage déterminée de températures, au moins un orifice de passage (3) ménagé dans la paroi de l'enceinte et muni d'un dispositif de fermeture amovible et un support (8) de récipients disposé à l'intérieur de l'enceinte et susceptible de permettre l'introduction et l'extraction un à un des récipients (7) ainsi que le cheminement pas à pas de ces derniers à l'intérieur de l'enceinte de manière qu'à chaque introduction d'un nouveau récipient corresponde l'extraction de celui des récipients ayant séjourné le plus longtemps dans l'enceinte.

2. Dispositif suivant la revendication 1, caractérisé en ce que lesdits moyens de chauffage (5) sont constitués par une ou plusieurs résistances électriques branchées en permanence sur une source de courant appropriée par l'intermédiaire desdits moyens de régulation (6), lesquels assurent en permanence à l'intérieur de ladite enceinte (1) une température demeurant entre 100 et 110°C environ de façon à avoir à l'intérieur des récipients une température légèrement inférieure à 100°C.

3. Dispositif suivant la revendication 1 ou 2, caractérisé en ce que ledit support de récipient (7) est constitué par un panier-magasin (8) cylindrique et d'axe vertical (10).

4. Dispositif suivant la revendication 3, caractérisé en ce que le panier-magasin (8) est constitué d'une grille de fond circulaire (11) et de cloisons rayonnantes (12) délimitant un certain nombre d'emplacements ou logements (9) de réception des biberons (7).

5. Dispositif suivant la revendication 4, caractérisé en ce que le panier-magasin (8) est monté rotatif à l'intérieur de l'enceinte (1), la commande de rotation s'effectuant depuis l'extérieur par tous moyens appropriés.

6. Dispositif suivant la revendication 5, caractérisé en ce que les moyens de commande de rotation du paniermagasin (8) sont constitués par une poignée, bouton, ou volant (13) extérieur à l'enceinte (1), disposé sur la face supérieure de celle-ci et solidaire en rotation dudit paniermagasin, lesdits moyens étant agencés de manière à ne permettre la rotation que dans un seul sens.

7. Dispositif suivant la revendication 1 ou 2, caractérisé en ce que ladite enceinte (1) est rectiligne et le support de récipients est un moyen de convoyage sans fin traversant d'un bout à l'autre ladite enceinte laquelle comporte un orifice d'entrée et un orifice de sortie aux deux extrémités de l'enceinte.

8. Dispositif suivant la revendication 1 ou 2, caractérisé en ce que ladite enceinte (1) est rectiligne, comporte à ses deux extrémités un orifice d'entrée et un orifice de sortie et est munie intérieurement d'un chemin de guidage fixe canalisant les uns derrière les autres les récipients.

0096138

FIG . 1

FIG. 2

0096138

# Office européen des brevets

## RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | A 61 L 2/06 |
| A | FR-A-2 150 858 (SCHIEDAMSCHE WERKTUIGEN EN MASCHINEFABRIEK) * revendications; page 6, lignes 38 à 40 * | 1 | A 23 L 3/02 |
| | --- | | |
| A | FR-A-1 266 869 (HOECHST) * résumé * | 1 | |
| | --- | | |
| A | FR-A- 541 897 (NIELSEN) * en entier * | 1 | |
| | ----- | | |

**DOMAINES TECHNIQUES RECHERCHES (Int. Cl. ³)**

A 61 L
A 23 L

Le présent rapport de recherche a été établi pour toutes les revendications

| Lieu de la recherche | Date d'achèvement de la recherche | Examinateur |
|---|---|---|
| LA HAYE | 08-02-1983 | MALHERBE Y.J.M. |